## Europäisches Patentamt

## European Patent Office

## Office européen des brevets

(11) Veröffentlichungsnummer: **0 528 238 B1**

(12) **EUROPÄISCHE PATENTSCHRIFT**

(45) Veröffentlichungstag der Patentschrift: **08.11.95**

(51) Int. Cl.6: **A61M 1/38**

(21) Anmeldenummer: **92113143.9**

(22) Anmeldetag: **01.08.92**

(54) **Separationseinrichtung zur Trennung von Blut in seine Bestandteile.**

(30) Priorität: **09.08.91 DE 4126341**

(43) Veröffentlichungstag der Anmeldung:
**24.02.93 Patentblatt 93/08**

(45) Bekanntmachung des Hinweises auf die
Patenterteilung:
**08.11.95 Patentblatt 95/45**

(84) Benannte Vertragsstaaten:
**AT CH ES FR GB IT LI NL**

(56) Entgegenhaltungen:
**EP-A- 0 089 003**
**EP-A- 0 097 366**
**EP-A- 0 155 684**
**US-A- 3 957 197**

**PATENT ABSTRACTS OF JAPAN vol. 13, no.
124 (C-580)27. März 1989 & JP-A-63294866
(IRYO KOGAKU KENKYUSHO KK)**

(73) Patentinhaber: **Fresenius AG
Gluckensteinweg 5
D-61350 Bad Homburg (DE)**

(72) Erfinder: **Biesel, Wolfgang Dr.
Henri-Dunant-Weg 3
W-6682 Ottweiler (DE)**

(74) Vertreter: **Dr. Fuchs, Dr. Luderschmidt Dr.
Mehler, Dipl.-Ing. Weiss Patentanwälte
Postfach 46 60
D-65036 Wiesbaden (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf eine Separationseinrichtung zur Trennung von Blut in seine Bestandteile im Rahmen einer in-vivo-Blutaufbereitung, mit einem Separator, der eine Zuführungsleitung mit einer Pumpe für die im Durchfluß einstellbare Zufuhr des zu trennenden Blutes und Abführungsleitungen für das getrennte Austragen mindestens der Erythrozytenfraktion und des zellarmen Plasmas sowie eine Rückführleitung aufweist, über die zumindest ein Bestandteil dem Spender/Patienten rückführbar ist, und mit einer Stellanordnung, der ein dem Hämatokritwert des zugeführten Blutes repräsentierendes Signal (Hämatokrit-Signal) aufgeschaltet ist.

Eine derartige Separationseinrichtung, die insbesondere bei der intraoperativen Blutaufbereitung Verwendung findet, ist durch die US-PS 39 57 197 bekannt geworden. Im bekannten Fall wird das Hämatokrit-Signal auf eine Regelstufe geschaltet, mit der der Hämatokritwert des Vollblutes (Anteil der Erythrozyten am Volumen des peripheren Blutes in Vol-%) auf einem konstanten Wert gehalten wird. Dazu wird der Hämatokritwert des Vollblutes in der Zuführungsleitung gemessen (Istwert) und mit einem Sollwert verglichen. Liegt der gemessene Hämatokritwert über dem Sollwert, wird er durch Hinzufügen einer abgetrennten Fraktion aus den entsprechenden Abführungsleitungen über eine exakt dosierende Pumpe zur Zuführungsleitung auf den vorgegebenen Wert einreguliert. Mit dieser Maßnahme kann das Verhältnis Plasmafluss zu Erythrozytenfluss auf einen konstanten, definierten Wert eingestellt und damit eine Wirkungsgradverbesserung der Separationseinrichtung hinsichtlich der Zellgewinnung bei größerer Blutschonung erreicht werden.

Vorrichtungen mit verbessertem Wirkungsgrad bei der Blutgewinnung und größerer Blutschonung gehen auch aus der DE 34 10 286 A1 (gleich EP 0 155 684 A2) hervor. Die DE 34 10 286 A1 beschreibt eine Vorrichtung, bei der der Hämatokrit des einer Zentrifuge zugeführten Vollblutes durch Plasmarückführung in den gewünschten niedrigen Bereich eingestellt wird (z.B. 25 %), bei der die Zentrifuge die optimale Trennleistung erzielt. Bei einem Eingangswert von 25 % Erythrozyten im Vollblut trennt die Zentrifuge bei konstantem Eingangsfluß und konstanter Drehzahl das Blut am schnellsten; der Endwert des Hämatokrit des Zellkonzentrates, i.e. der Erythrozytenfraktion (z.B. 70 %), wird am schnellsten erreicht. Aus diesem Grund wird das bei der Zentrifugation gewonnene Plasma zugeführt und damit das Blut verdünnt.

Ein entsprechender Stand der Technik mit Regelung des Hämatokritwertes des zugeführten Vollblutes auf einen konstanten Wert ist durch den Aufsatz von B.J. Van Wie und S. S. Sofer, The effect of recycle on the continuous centrifugal processing of blood cells, The International Journal of Artificial Organs, Vol. 8, no. 1, 1985, Seiten 43-48 bekannt geworden.

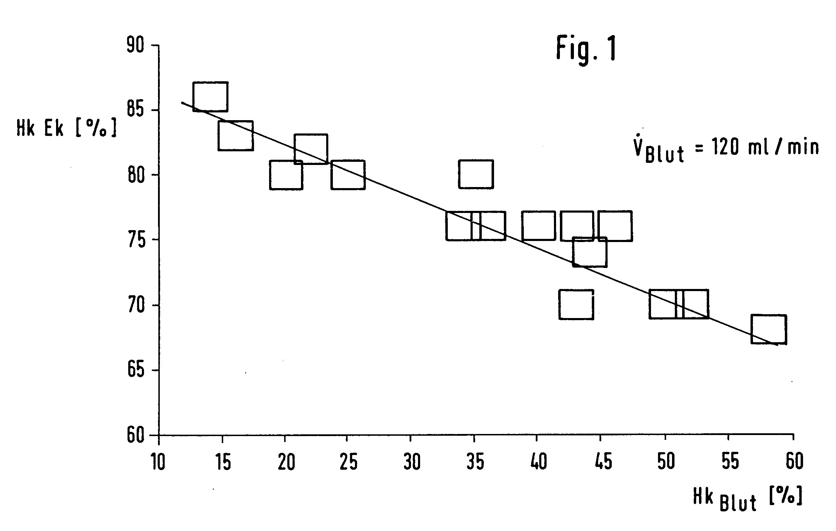

Bei bestimmten medizinischen Anwendungfällen, insbesondere bei der intraoperativen Blutaufbereitung, muß man jedoch von regeltechnisch nicht beeinflußbaren, breit streuenden, d.h. von stark verschieden hohen Werten des Hämatokrit des einer Separationseinrichtung zugeführten Vollblutes ausgehen. Die bekannten Vorrichtungen sind dann nicht einsetzbar. Der Hämatokritwert des eintretenden Blutes spielt jedoch hinsichtlich der Sedimentation zellulärer Blutbestandteile in einer Zentrifuge eine erhebliche Rolle, da die Sedimentation außer von der Größe, der Dichte und der Form der Blutzellen auch vom Hämatokritwert abhängig ist. Dies hat zur Folge, daß eintretendes Blut, welches unterschiedliche Hämatokritwerte aufweist, bei gleicher Verweildauer in einer Zentrifuge zu unterschiedlich hoch konzentrierten Zellfraktionen separiert wird. Dieser Zusammenhang zwischen dem Hämatokritwert des Blutes ( = Hk Blut in %) und dem Hämatokritwert der Erythrozytenfraktion (HkEk in %) bei einem konstanten Blutfluß von 120 ml/min ist in Figur 1 dargestellt. Mit wachsendem Hk Blut nimmt der HkEk ab.

Ähnlich liegen die Verhältnisse bei anderen Separationseinrichtungen, insbesondere Filtrationssystemen, die ebenfalls eingesetzt werden können.

Die in Figur 1 dargestellte Abhängigkeit ist bei solchen typischen Anwendungsfällen von Zellseparatoren ohne besondere Bedeutung, wo ausreichende Behandlungszeiträume in den Zentrifugen realisiert werden können und wo möglicherweise auch ein schlechterer Wirkungsgrad der Separation in Kauf genommen werden kann. Weiterhin weist der Eingangs-Hämatokritwert des Spenderblutes im Durchschnitt nur geringe Schwankungen auf, so daß die diesbezüglichen Effekte auch in derartigen Fällen vernachlässigbar sind.

Anders ist es jedoch besonders im Bereich der intraoperativen Autotransfusion, insbesondere bei Plasmaseparations- und Waschverfahren. Dort spielt der Hämatokritwert eine entscheidende Rolle. Intraoperativ anfallendes, im Operationsfeld abgesaugtes und in einem Sammelbehältnis zwischengelagertes Blut weist in Abhängigkeit von der Art der Operation, dem Hämatokritwert des Patienten, der Menge der im Operationsfeld eingesetzten Spüllösung, der Menge infundierte Volumenexpander, dem Blutverlust des Patienten, der Menge des zugeführten Antikoagulans oder der Menge eingeschwemmter Flüssigkeit, beispielsweise aus dem Gewebe, sehr unterschiedliche Hämatokritwerte auf. Diese liegen im Bereich von zehn bis vierzig Prozent und können sich während einer Operation beliebig ändern. Demgegenüber liegen die Normalwerte für Männer bei sechsundvierzig und für Frauen bei vierzig Volumenprozent. Die derzeit üblichen Aufbereitungsverfahren berücksichtigen diese Schwankungen des Hämatokritwertes nicht und arbeiten üblicherweise mit konstanter, voreingestellter Blutförderrate in die Zentrifuge. Dies führt zu folgenden Konsequenzen:

1. Der Hämatokritwert des produzierten Erythrozytenkonzentrates unterliegt großen Schwankungen.

2. Die Plasmaauswaschrate, welche durch das Volumenverhältnis von auszuwaschendem, im Zellkonzentrat verbliebenem Restplasma zur zugeführten Waschlösung definiert ist, unterliegt erheblichen Schwankungen.

3. Die Sedimentationskapazität der Separationseinrichtung kann nicht optimal genutzt werden.

Weiterhin ist ein konstanter Hämatokritwert des produzierten Erythrozytenkonzentrates zur exakten Dokumentation der retransfundierten Erythrozytenmenge über das retransfundierte Erythrozytenkonzentratvolumen unerläßlich. Es ist wünschenswert, mit einer möglichst hohen Blutaufbereitungsgeschwindigkeit und mit einer konstanten Auswaschrate zu arbeiten.

Der Erfindung liegt die Aufgabe zugrunde, ausgehend von der eingangs bezeichneten Separationseinrichtung diese in der Steueranordnung so auszubilden, daß trotz stark schwankendem Hämatokritwert des zugeführten Blutes ein konstanter Hämatokritwert des aufbereiteten Zellkonzentrates erzielbar ist. Weitere Ziele liegen in einer konstanten Auswaschrate und der optimalen Ausnutzung der Sedimentationsleistung der verwendeten Separationseinrichtung zur Optimierung des Blutdurchsatzes.

Die Lösung dieser Aufgabe gelingt gemäß der Erfindung dadurch, daß die Stellanordnung eine Steuerstufe mit vorgegebener Übertragungsfunktion aufweist, deren Eingang das Hämatokrit-Signal zugeführt und deren Ausgang mit der Pumpe (16) in der Zuführungsleitung verbunden ist, und daß die Übertragungsfunktion so bestimmt ist, daß abhängig von der Größe des Hämatokrit-Signals ein Verstellsignal am Ausgang der Steuerstufe ableitbar ist, das den Blutfluß durch die Pumpe (16) in den Separator so einstellt, daß der Hämatokritwert der rückgeführten Erythrozytenfraktion einen konstanten Wert hat.

Die Erfindung bietet somit die Vorteile, daß trotz eines breit streuenden Hämatokritwertes im zugeführten Blut - im Bereich der intraoperativen Autotransfusion treten Schwankungen im Bereich von ca. 10 - 40 % auf - durch Steuerung des Blutflusses und damit veränderter Separationszeit dennoch ein konstanter Hämatokritwert des Zellkonzentrats erzielbar ist. Die Trennzeit ist dabei optimal kurz, d.h. die Leistung des Systems ist gleichzeitig hinsichtlich der Aufbereitungskapazität optimiert.

Ausgestaltungen der Erfindung sind in den Unteransprüchen der Patentansprüche beschrieben.

Im Folgenden wird die Erfindung anhand von Ausführungsbeispielen, die in den Zeichnungen dargestellt sind, beschrieben. Dabei zeigt:

Fig. 1.   eine Darstellung der Abhängigkeit des Hämatokritwertes Hk der Erythrozytenfraktion Ek von dem Hk des zugeführten Blutes bei konstantem Durchfluß,

Fig. 2   eine schematische Block-Darstellung einer erfindungsgemäßen Separationseinrichtung,

Fig. 3   eine Darstellung der Abhängigkeit des Hämatokritwertes des zu trennenden Blutes von dem Blutfluß bei konstantem Hämatokritwert der Zellfraktion und

Fig. 4   eine Blockdiagramm-Darstellung eines weiteren Ausführungsbeispieles einer erfindungsgemäßen Separationseinrichtung.

Die jeweiligen Ausführungsbeispiele beziehen sich auf Separatoren in Form einer Blutzentrifuge. Es können jedoch auch andere Separatoren, z.B. Filtrationssysteme, verwendet werden, die die Blutzellen von der Flüssigkeit trennen.

Wenn im folgenden von Blut gesprochen wird, sollen damit ganz allgemein blutzellenhaltige Suspensionen verstanden werden, also auch Blut/Lösungsgemische.

Die Figur 1 zeigt den Zusammenhang zwischen dem Hämatokritwert Hk des zu trennenden Blutes und dem des Zellkonzentrates Ek bei vorgegebenem Blutfluß $\dot{V}_B$ von 120 ml/min. Es ist dabei deutlich sichtbar, daß Blut mit unterschiedlichem Hämatokritwert bei gleicher Verweildauer in einer Zentrifuge, d.h. bei gleichbleibender Fördergeschwindigkeit, zu unterschiedlich hoch konzentrierten Zellfraktionen Ek separiert wird. Je größer der Hk Blut ist, umso kleiner ist der HkEk. Die Figur 1 zeigt somit den Zustand, welcher aus dem Stand der Technik bekannt ist.

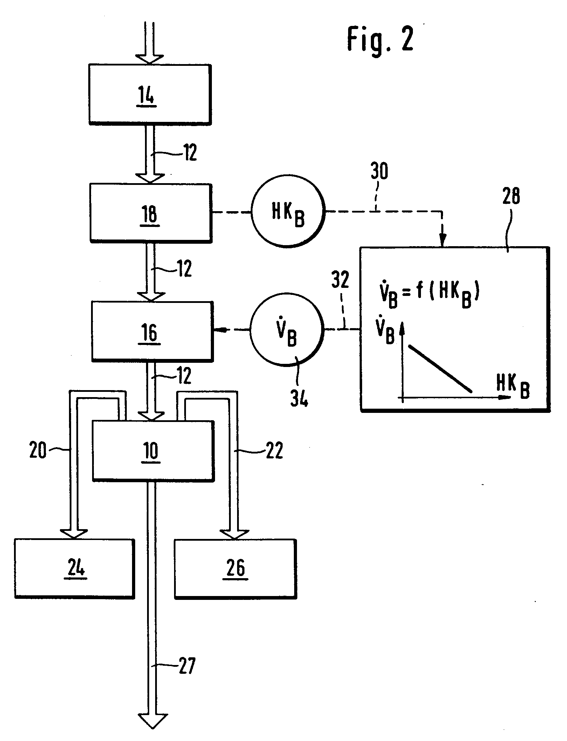

Die Figur 2 zeigt eine Zentrifuge 10 als Separator, die eine Zuführungsleitung 12 für die Zufuhr des zu trennenden bzw. aufzubereitenden, in einer Blutquelle 14 befindlichen Blutes aufweist. In diese Zuführungsleitung sind eine in der Drehzahl einstellbare und damit den Blutdurchfluß veränderbare Blutpumpe 16 sowie stromauf dieser Pumpe ein Hämatokritsensor 18 eingeschaltet. Mit diesem Sensor 18 wird der Hämatokritwert des zugeführten Blutes $Hk_B$ mit bekannten Methoden, z.B. fotoelektrisch oder mittels einer Leitfähigkeitsmessung, ermittelt. Auch die anderen Komponenten 10 bis 16 sind bekannte Baugruppen, so daß sich eine nähere Beschreibung dieser Gruppen erübrigt.

Die Zentrifuge 10 weist ferner Abführungsleitungen 20 und 22 für das getrennte Austragen der Blutbestandteile auf, wobei in Figur 2 nur zwei Bestandteile, die Erythrozytenfraktion und das zellarme Plasma in zugehörigen Behältnissen 24 und 26 gesammelt werden. Der Einfachheit und Übersichtlichkeit halber sind in Figur 2 auch alle weiteren üblichen Bestandteile des Separationssystemes weggelassen, z.B. die in-vivo-Blutentnahme und auch die üblichen Pumpen in den Abführungsleitungen. Die Rückführleitung mit Zellkonzentrat zum Patienten/Spender ist gemäß Fig. 2 mit 27 bezeichnet.

In Figur 2 ist weiterhin eine Steuerstufe 28 dargestellt, deren Eingang das den Hämatokritwert des zugeführten Blutes repräsentierende Signal, das Hämatokritsignal $Hk_B$ über die Leitung 30 zugeführt ist und deren Ausgang über die Leitung 32 mit der Verstelleinrichtung 34 der Pumpengeschwindigkeit an der Blutpumpe 16 und damit des Blutdurchflusses $\dot{V}_B$ verbunden ist. Die Steuerstufe 28 kann aus bekannten analogen Steuerkreisen aufgebaut werden. Sie wird vorzugsweise durch einen Mikroprozessor gebildet. Die Übertragungsfunktion der Steuerstufe 28 ist dabei so bestimmt, daß abhängig von der Größe des Eingangssignals $Hk_B$ am Ausgang der Steuerstufe 28 ein Verstellsignal $\dot{V}_B$ ableitbar ist, welches die Flußrate des Blutes in die Zentrifuge 10 so einstellt, daß der Hämatokritwert unter Berücksichtigung der für die jeweilige Separationskammer typischen zeitlichen Verzögerung, hier der dem Patienten rückgeführten Erythrozytenfraktion, einen konstanten Wert hat. Die Vorgabe der Übertragungsfunktion $\dot{V}_B = f(Hk_B)$ in der Steuerstufe 28 erfolgt anhand empirisch oder rechnerisch ermittelter Kennliniendiagramme.

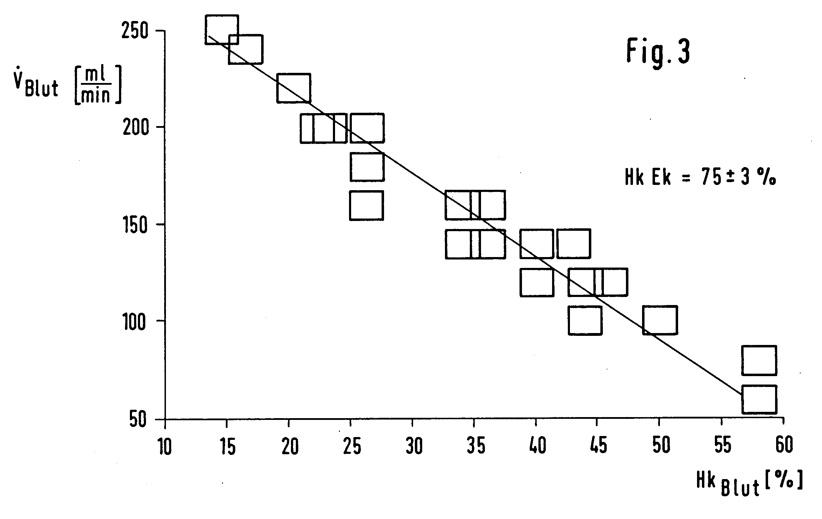

Die Figur 3 zeigt ein Beispiel für ein derartiges Kennliniendiagramm, welches den Zusammenhang zwischen Eingangs-Hämatokritwert $Hk_B$ und Blutfluß $\dot{V}_B$ bei konstantem Hämatokrit der Erythrozytenfraktion $HkEk = 75\%$ darstellt. Zu jedem gemessenen $Hk_B$ gibt es einen Wert $\dot{V}_B$, bei dem der vorgegebene $HkEk$ erreicht wird. Diese Maßnahme gemäß Figur 3 mit kontinuierlicher Messung des Hämatokritwertes $Hk_B$ ist insbesondere geeignet zur Verwendung bei Zentrifugen mit kontinuierlicher und diskontinuierlicher Verfahrensweise (Blutdurchfluß-Zentrifuge bzw. Batch-Verfahren).

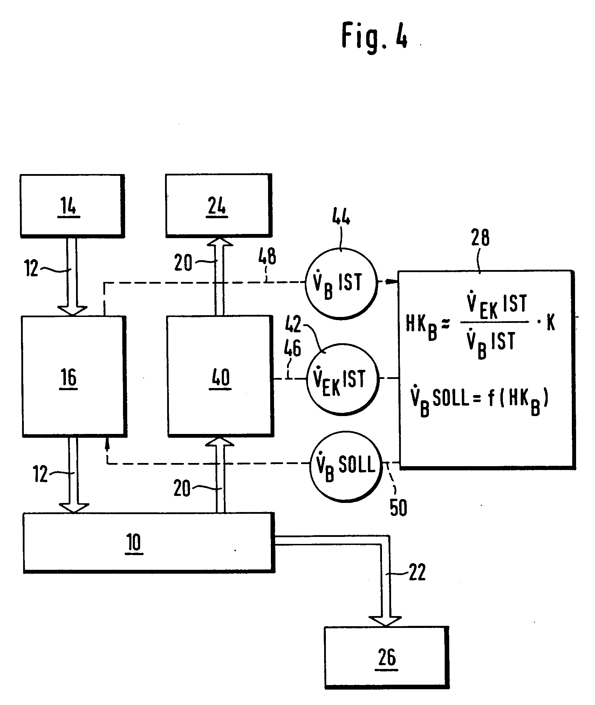

Bei der Einrichtung nach Figur 2 wird der Hämatokritwert des Blutes direkt und kontinuierlich gemessen. Die Figur 4 zeigt eine alternative Ausgestaltung einer erfindungsgemäßen Vorrichtung, bei welcher die Hämatokrit-Bestimmung indirekt erfolgt. Eine derartige Vorgehensweise ist insbesondere bei Blutdurchflußzentrifugen mit Einrichtungen zur Bilanzierung der in die Separationskammer und aus der Separationskammer fließenden Teilströme (Blut, Plasma, Waschlösung, Erythrozytenkonzentrat) verwendbar. Die Hämatokrit-Bestimmung erfolgt dabei näherungsweise rechnerisch aus dem Blutfluß in die Separationskammer und dem Erythrozyten-Konzentratfluß bzw. Plasmafluß aus der Separationskammer nach einer der beiden nachfolgenden Formeln.

a)

$$Hk_B = \frac{\dot{V}_{EK}}{\dot{V}_B} * K$$

oder

b)

$$Hk_B = \frac{\dot{V}_B - \dot{V}_{PLS}}{\dot{V}_B} * K$$

| | |
|---|---|
| $Hk_B =$ | Hk des Blutes |
| $K =$ | Konstante |
| $\dot{V}_{EK} =$ | Erythrozytenkonzentratfluß |
| $\dot{V}_B =$ | Blutfluß |
| $\dot{V}_{PLS} =$ | Plasmafluß |

Die Konstante K entspricht dem gewünschten, für das Kennliniendiagramm gemäß Fig. 3 typischen Hämatokritwert des Erythrozytenkonzentrates, beispielsweise einem Wert von 75% (Zielhämatokrit).

In der Figur 4 ist die Alternative a) dargestellt. Alle Teile der Einrichtung nach Figur 4, die mit derjenigen nach Figur 2 übereinstimmen, haben dieselben Bezugsziffern. Zusätzlich dargestellt sind die Pumpe 40 in der Abführungsleitung 20 der Erythrozytenfraktion, der Sensor 42 für die Gewinnung des Meßwertes betreffend den Erythrozytenkonzentratfluß ($\dot{V}$EK ist) und der Sensor 44 für die Gewinnung des Meßwertes betreffend den Wert des Blutflusses ($\dot{V}$B ist) in die Zentrifuge 10. Dabei sind der Sensor 42 über die Leitung 46 mit der Ek-Pumpe 40 und der Sensor 44 über die Leitung 48 mit der Blutpumpe 16 verbunden.

Die Steuerstufe 28' ist gegenüber der Steuerstufe 28 von Figur 2 erweitert. An der Steuerstufe 28' steht das Hämatokritsignal $Hk_B$ nicht mehr unmittelbar an, sondern wird quasi in einer weiteren vorgeschalteten Stufe erst gebildet. Wenn die Steuerstufe 28' durch einen Mikroprozessor bebildet wird, sind die Stufenunterschiede nicht mehr hardwaremäßig ausgebildet, sondern machen sich im Programm bemerkbar. Die Umrandungen der Stufen 28 in Figur 2 sowie 28' in Figur 4 sind daher funktional zu sehen und nicht etwa als Gehäusewandung.

Die Einstellung des Blutflusses an der Blutpumpe 16 erfolgt ausgehend von einem vorgegebenem Wert $V_B$, der einen mittleren Hämatokritwert des Erythrozytenkonzentrats von beispielsweise 75 ± 10 % über den gesamten Hämatokritbereich im zugeführten Blut gewährleistet. Dabei ist der Ausgang der Steuerstufe 28' über eine Leitung 50 mit dem Eingang der Blutpumpe 16 verbunden, auf den der einzustellende Wert $V_B$ SOLL geschaltet wird. Nach Einstellung eines Gleichgewichtszustandes erfolgt die Bestimmung des Wertes $V_{EK}$ bzw. die Berechnung des Hämatokritwertes des zugeführten Blutes und die entsprechende Anpassung des Blutflusses. Ein derartiger Gleichgewichtszustand ist beispielsweise durch ein dem Hämatokrit des zugeführten Blutes entsprechendes Verhältnis von Blutfluß und Erythrozytenkonzentratfluß bzw. Plasmafluß und damit durch einen konstanten Füllzustand der Zentrifuge gekennzeichnet. Die Abweichung des berechneten Hämatokritwertes vom tatsächlichen Hämatokritwert ist abhängig von der Differenz zwischen dem tatsächlich eingestellten Blutfluß $V_B$ IST und dem gemäß Fig. 3 einzustellenden Blutfluß VBlut. Durch schrittweise Anpassung des Blutflusses entsprechend dem bestimmten Hämatokritwert vermindert sich die Differenz zwischen eingestelltem und einzustellendem Blutfluß und somit auch der Fehler der Hämatokritberechnung. Dies ergibt sich aus nachfolgendem Beispiel einer hämatokritorientierten Blutflußeinstellung.

Randbedingungen:

- gewünschter Hämatokrit des Erythrozytenkonzentrats $Hk_{EK}$ = 75 %
- maximaler Blutfluß $V_B$, der auch im hohen $Hk_B$-Bereich einen $Hk_{EK}$ größer 70 % ermöglicht = 120 ml/min
- aufzubereitendes Blut mit $Hk_B$ = 20 %
- Separationskammer PL1 bei 2.000/Umin

Einstellvorgang (1. Rechenbeispiel):

Startbedingung: voreingestellter Blutfluß $\dot{V}_B$ = 120 ml/min führt zu einem Konzentratfluß $\dot{V}_{EK}$ von 29 ml/min mit $Hk_{EK}$ = 83 %;

1. Einstellschritt:

$$\text{berechneter } Hk_B = \frac{29}{120} * 75 = 18,2 \text{ \%;}$$

Einstellung von $\dot{V}_B$ (18,2) nach Fig. 3 = 220 ml/min → Konzentratfluß $\dot{V}_{EK}$ = 60 ml/min bei $Hk_{EK}$ = 72 %

2. Einstellschritt:

$$\text{neu berechneter } Hk_B = \frac{60}{220} * 75 = 20{,}5\ \%;$$

Einstellung von $\dot{V}_B$ (20,5) nach Fig. 3 = 200 ml/min → Konzentratfluß $\dot{V}_{EK}$ = 56 ml/min bei $Hk_{EK}$ = 75 %

Bei der erfindungsgemäßen Steuerung nach Figur 4 erfolgt somit ein selbsttätiges Einstellen des gewünschten $Hk_{EK}$ von 75 % bei maximalem Konzentratfluß. Das ungesteuerte System verbliebe unter den als Startbedingung bezeichneten Bedingungen und produzierte Erythrozytenkonzentrat von $Hk_{EK}$ = 83 % bei einem Konzentratfluß von 29 ml/min. Anstelle der beschriebenen hämatokrit-orientierten Blutflußeinstellung ist auch eine am Erythrozytenkonzentrat(EK)-Fluß orientierte Einstellung des Blutflusses möglich, denn der Hämatokrit kann nach Gleichung $V_{EK}$ ist = f ($Hk_B$) durch den Erythrozyten-Konzentratfluß $\dot{V}_{EK}$ repräsentiert werden. Nachfolgendes Rechenbeispiel verdeutlicht die EK-Fluß orientierte Blutflußeinstellung:

Rechenbeispiel 2:

Startbedingung: Soll-EK-Fluß = 56 ml/min ($= \dot{V}_{EK}$soll) ($Hk_{EK}$ = 75 %) Voreingestellter Blutfluß $V_B$ = 120 ml/min führt zu einem $\dot{V}_{EK}$ ist von 29 ml/min ($Hk_{EK}$ . 83 %)

1. Einstellschritt:

$$\frac{\dot{V}_{EK} \text{ soll}}{\dot{V}_{EK} \text{ ist}} = \frac{56 \text{ ml/min}}{29 \text{ ml/min}} = 1{,}93$$

Einstellung $\dot{V}_3$ soll = $\dot{V}_B$ ist . 1,93 = 120 ml/min. 1,93 $\cong$ 232 ml/min

2. Einstellschritt:

$\dot{V}_B$ = 232 ml/min führt zu einem $\dot{V}_{EK}$ ist = 65 ml/min ($Hk_{EK}$ = 70%)

$$\frac{\dot{V}_{EK} \text{ soll}}{\dot{V}_{EK} \text{ ist}} = \frac{56 \text{ ml/min}}{65 \text{ ml/min}} = 0{,}86$$

Einstellung $\dot{V}_B$ soll = - $\dot{V}_B$ ist . 0.86 = 232 ml/min . 0.86 $\cong$ 200 ml/min ($\dot{V}_B$ soll) von 200 ml/min führt zu einem $\dot{V}_{EK}$ = 56 ml/min ($Hk_{EK}$ =75%)

In der nachfolgenden Tabelle sind die Werte $\dot{V}_{EK}$ und $Hk_{EK}$ für Hämatokritwerte des eintretenden Blutes von 10 - 40 % im gesteuerten und ungesteuerten System vergleichsweise gegenüber gestellt.

| $Hk_B$ | gesteuert | | ungesteuert | |
|---|---|---|---|---|
| | $\dot{V}_{EK}$ | $Hk_{EK}$ | $\dot{V}_{EK}$ | $Hk_{EK}$ |
| % | ml/min | % | ml/min | % |
| 10 | 53 | 75 | 14 | 90 ca. |
| 20 | 56 | 75 | 29 | 83 |
| 30 | 56 | 75 | 48 | 78 |
| 40 | 54 | 75 | 64 | 73 |

Man erkennt deutlich die unerwünschte Schwankungsbreite des Hämatokritwertes des Erythrozytenkonzentrats $Hk_{EK}$ von 73 - 90 % im ungesteuerten Fall gegenüber dem konstanten Wert von 75 % im gesteuerten Fall.

Die Erfindung ist nicht auf die gezeigten Ausführungsbeispiele beschränkt, vielmehr ergeben sich im Rahmen der Erfindung vielfältige Abwandlungs- und Modifikationsmöglichkeiten.

**Patentansprüche**

1. Separationseinrichtung zur Trennung von Blut in seine Bestandteile im Rahmen einer in-vivo-Blutaufbereitung, insb. bei der intraoperativen Blutaufbereitung, mit einem Separator (10), der eine Zuführungsleitung (12) mit einer Pumpe (16) für die im Durchfluß einstellbare Zufuhr des zu trennenden Blutes und Abführungsleitungen (20, 22) für das getrennte Austragen mindestens der Erythrozytenfraktion und des zellarmen Plasmas sowie eine Rückführleitung (27) aufweist, über die zumindest ein Bestandteil dem Spender/Patienten rückführbar ist, und mit einer Stellanordnung, der ein den Hämatokritwert des zugeführten Blutes repräsentierendes Signal (Hämatokrit-Signal) aufgeschaltet ist, dadurch gekennzeichnet, daß die Stellanordnung eine Steuerstufe (28, 28') mit vorgegebener Übertragungsfunktion aufweist, deren Eingang (30) das Hämatokrit-Signal zugeführt und deren Ausgang (32) mit der Pumpe (16) in der Zuführungsleitung (12) verbunden ist, und daß die Übertragungsfunktion so bestimmt ist, daß abhängig von der Größe des Hämatokrit-Signals ein Verstellsignal (34) am Ausgang der Steuerstufe (28) ableitbar ist, das den Blutfluß durch die Pumpe (16) in den Separator (10) so einstellt, daß der Hämatokritwert der rückgeführten Erythrozytenfraktion einen konstanten Wert hat.

2. Einrichtung nach Anspruch 1, dadurch gekennzeichnet, daß in der Zuführungsleitung (12) ein Sensor (18) zur kontinuierlichen Messung des Hämatokritwertes des zu trennenden Blutes vorgesehen ist, der das Hämatokrit-Signal abgibt.

3. Einrichtung nach Anspruch 1, dadurch gekennzeichnet, daß ein Sensor (44) zum Ableiten eines Signals über den Blutdurchfluß in den Separator (10) und ausgangsseitig des Separators (10) ein Sensor (42) zur Bestimmung des Durchflusses der Erythrozytenfraktion oder ein Sensor zur Bestimmung des Durchflusses der Plasmafraktion vorgesehen sind, und daß in der Steuerstufe (28') eine vorgeschaltete Rechenstufe vorgesehen ist, der die Durchflußsignale zugeführt sind und die daraus den Hämatokritwert des zu trennenden Blutes errechnet.

4. Einrichtung nach Anspruch 3, dadurch gekennzeichnet, daß die Berechnung des Hämatokritwertes in der Rechenstufe nach folgender Beziehung erfolgt:

$$Hk_B = \frac{\dot{V}_{EK}}{\dot{V}_B} * K$$

mit

$Hk_B$ = Hk des Blutes
$K$ = Konstante (Zielhämatokrit)
$\dot{V}_{EK}$ = Erythrozytenkonzentratfluß
$\dot{V}_B$ = Blutfluß

5. Einrichtung nach Anspruch 3, dadurch gekennzeichnet, daß die Berechnung des Hämatokritwertes in der Rechenstufe nach folgender Beziehung erfolgt:

$$Hk_B = \frac{\dot{V}_B - \dot{V}_{PLS}}{\dot{V}_B} * K$$

7

mit

$Hk_B$      = Hk des Blutes

K      = Konstante (Zielhämatokrit)

$\dot{V}_B$      = Blutfluß

$\dot{V}_{PLS}$      = Plasmafluß

6. Einrichtung nach einem der Ansprüche 3 - 5, dadurch gekennzeichnet, daß die Hämatokritbestimmung in der Rechenstufe und die Anpassung des Blutflusses iterativ nach einem Näherungsverfahren erfolgt

7. Einrichtung nach Anspruch 6, dadurch gekennzeichnet, daß die Einstellschritte für die Anpassung des Blutflusses Hämatokrit- oder alternativ Erythrozytenkonzentratfluß-orientiert sind.

8. Einrichtung nach einem der Ansprüche 1 - 7, dadurch gekennzeichnet, daß die Steuerstufe einschl. Rechenstufe (28') durch einen Mikroprozessor gebildet ist.

9. Einrichtung nach einem der Ansprüche 1 - 8, dadurch gekennzeichnet, daß der Separator (10) eine Zentrifuge ist.

## Claims

1. Apparatus for separation of blood into its components within the scope of an in-vico-blood processing, in particular the intraoperative blood processing, comprising a separator (10) which has one intake line (12) with a pump (16) whose flow can be adjusted to supply the blood to be separated and discharge lines (20, 22) for the separated output of at least the erythrocyte fraction and the cell-poor plasma as well as a return line (27), via which at least one component can be returned to the donor/patient, and a control arrangement to which a signal representing the hematocrit value of the incoming blood (hematocrit signal) is sent, characterized in that the control arrangement comprising a regulating means (28, 28') with a preset transmission function, whose input (30) delivers the hematocrit signal and whose output (32) is linked with the pump (16) in the intake line (12) and that the transmission function is specified such that, depending on the magnitude of the hematocrit signal, an adjustment signal (34) can be derived at the output of the regulating step (28), which adjusts the blood flow through the pump (16) into the separator (10) such that the hematocrit value of the erythrocyte fraction returned has a constant value.

2. The apparatus according to claim 1, characterized in that a sensor (18) for continuous measurement of the hematocrit value is provided in the intake line (12), which sensor delivers the hematocrit signal.

3. The apparatus according to claim 1, characterized in that a sensor (44) is provided to derive a signal reporting the blood flow into the separator (10), and on the output side of the separator (10) a sensor (42) to determine the flow of the erythrocyte fraction or a sensor to determine the flow of the plasma fraction is provided, and that in the regulating means (28') an upstream calculation step is provided, to which the flow signals are delivered and which calculates the hematocrit value of the blood to be separated therefrom.

4. The apparatus according to claim 3, characterized in that the calculation of the hematocrit value in the calculation step occurs according to the following formula:

$$Hk_B = \frac{\dot{V}_{EK}}{\dot{V}_B} * K$$

wherein

$Hk_B$ =      Hk of the blood

K =      constant (target hematocrit)

$\dot{V}_{EK}$ =     erythrocyte concentrate flow
$\dot{V}_{B}$ =     blood flow

5. The apparatus according to claim 3, characterized in that the calculation of the hematocrit value in the calculation step occurs according to the following formula:

$$Hk_B = \frac{\dot{V}_B - \dot{V}_{PLS}}{\dot{V}_B} * K$$

wherein
$Hk_B$ =     Hk of the blood
K =     constant (target hematocrit)
$\dot{V}_B$ =     blood flow
$\dot{V}_{PLS}$ =     plasma flow

6. The apparatus according to any of claims 3-5, characterized in that the hematocrit determination in the calculation step and the adjustment of the blood flow occurs iteratively according to an approximation process.

7. The apparatus according to claim 6, characterized in that the adjustment steps for the adaption of the blood flow are alternatively based on the hematocrit or the erythrocyte concentrate flow.

8. The apparatus according to any of claims 1-7, characterized in that the control arrangement including the regulating means (28') comprises a microprocessor.

9. The apparatus according to any of claims 1-8, characterized in that the separator (10) is a centrifuge.

**Revendications**

1. Dispositif de séparation du sang en ses composants dans le cadre d'un traitement sanguin in vivo, en particulier dans le cas du traitement sanguin peropératoire, comprenant un séparateur (10) qui présente une conduite d'amenée (12) avec une pompe (16) pour l'amenée, à débit variable, du sang à séparer, et des conduites d'évacuation (20, 22) pour l'enlèvement séparé d'au moins la fraction érythrocytaire et du plasma pauvre en cellules, ainsi qu'une conduite de retour (27) par le biais de laquelle au moins une partie peut être renvoyée au donneur/patient, et comprenant un dispositif de réglage sur lequel intervient un signal représentant la valeur hématocrite du sang amené (signal hématocrite), caractérisé en ce que le dispositif de réglage présente un étage de commande (28, 28') comprenant une fonction de transmission prédéfinie à l'entrée (30) de laquelle est conduit le signal hématocrite et dont la sortie (32) est reliée à la pompe (16) dans la conduite d'amenée (12), et en ce que la fonction de transmission est définie de telle façon que, en fonction de la valeur du signal hématocrite, un signal de réglage (34) peut être dérivé à la sortie de l'étage de commande (28), signal qui règle le débit sanguin à travers la pompe (16) dans le séparateur (10) de telle façon que la valeur hématocrite de la fraction érythrocyte renvoyée présente une valeur constante.

2. Dispositif selon la revendication 1, caractérisé en ce que, dans la conduite d'amenée (12), il est prévu un capteur (18) chargé de mesurer en continu la valeur hématocrite du sang à séparer, valeur qui est fournie par le signal hématocrite.

3. Dispositif selon la revendication 1, caractérisé en ce qu'il est prévu un capteur (44) pour la dérivation d'un signal concernant le débit sanguin dans le séparateur (10) et, un capteur (42) pour la détermination du débit de la fraction érythrocyte ou un capteur pour déterminer le débit de la fraction de plasma, sur le côté sortie du séparateur (10), et en ce que, dans l'étage de commande (28'), il est prévu un étage de calcul en amont duquel sont conduits les signaux de débit et qui calcule à partir de cela la

9

valeur hématocrite du sang à séparer.

4. Dispositif selon la revendication 3, caractérisé en ce que le calcul de la valeur hématocrite dans l'étage de calcul s'effectue selon la relation suivante :

$$Hk_B = \frac{\dot{V}_{EK}}{\dot{V}_B} * K$$

où

$Hk_B =$      Hk du sang
$K =$      constante (hématocrite visé)
$V_{EK} =$      débit de concentré érythrocytaire
$V_B =$      débit sanguin

5. Dispositif selon la revendication 3, caractérisé en ce que le calcul de la valeur hématocrite dans l'étage de calcul s'effectue de la façon suivante :

$$Hk_B = \frac{\dot{V}_B - \dot{V}_{PLS}}{\dot{V}_B} * K$$

où

$Hk_B =$      Hk du sang
$K =$      constante (hématocrite visé)
$V_B =$      débit sanguin
$V_{PLS} =$      débit plasmatique

6. Dispositif selon l'une des revendications 3 à 5, caractérisé en ce que la détermination hématocrite s'effectue dans l'étage de calcul, et en ce que l'adaptation du débit sanguin s'effectue de façon itérative selon une méthode par approximation.

7. Dispositif selon la revendication 6, caractérisé en ce que les pas de réglage pour l'adaptation du débit sanguin sont alignés sur le flux de concentré hématocrite ou bien sur le flux de concentré érythrocytaire.

8. Dispositif selon l'une des revendications 1 à 7, caractérisé en ce que l'étage de commande, y compris l'étage de calcul (28'), est constitué d'un microprocesseur.

9. Dispositif selon l'une des revendications 1 à 8, caractérisé en ce que le séparateur (10) est une centrifugeuse.

Fig. 1

$\dot{V}_{Blut} = 120 \ ml \ / \ min$

Hk Ek [%]

Hk $_{Blut}$ [%]

EP 0 528 238 B1

Fig. 2

$$\dot{V}_B = f(HK_B)$$

Fig.3

Hk Ek = 75 ± 3 %

EP 0 528 238 B1

# Fig. 4

$$HK_B \approx \frac{\dot{V}_{EK}\,IST}{\dot{V}_B\,IST} \cdot K$$

$$\dot{V}_B\,SOLL = f(HK_B)$$